# EUROPEAN PATENT SPECIFICATION

(11) **EP 1 551 469 B1**
(45) Date of publication and mention of the grant of the patent: **08.03.2006**
(21) Application number: 03760460.0
(22) Date of filing: 18.06.2003
(51) Int. Cl.: A61L 27/26, A61L 29/04

(54) **BIOACTIVE AGENT RELEASE COATING WITH AROMATIC POLY(METH)ACRYLATES**
BIOAKTIVE-WIRKSTOFFFREISETZENDE BESCHICHTUNG MIT AROMATISCHEN POLY(METH)ACRYLATEN
REVETEMENT LIBERANT DES AGENTS BIOACTIFS AVEC DES POLY(METH)ACRYLATES AROMATIQUES

(30) Priority: 18.06.2002 US 174635
(43) Date of publication of application: 13.07.2005
(73) Proprietor: SurModics, Inc., Eden Prairie, MN 55344 (US)
(72) Inventor: LAWIN, Laurie, R., New Brighton, MN 55112 (US); OFSTEAD, Ronald, F., Maplewood, MN 55117 (US); CHAPPA, Ralph, A., Prior Lake, MN 55372 (US); HERGENROTHER, Robert, W., Eden Prairie, MN 55347 (US); ANDERSON, Aron, B., Minnetonka, MN 55343 (US); TRAN, Linh, V., Brooklyn Park, MN 55445 (US)
(74) Representative: Walker, Ross Thomson
(86) International application number: PCT/US2003/019249
(87) International publication number: WO 2003/105918

(56) References cited:
- EP-A- 0 879 595
- EP-A- 1 174 157
- US-A1- 2002 026 236
- US-A1- 2002 032 434
- US-B1- 6 344 035
- RIGGS P D ET AL: "Chlorhexidine release from room temperature polymerising methacrylate systems - in vitro studies with hydrocortisone" BIOMATERIALS, ELSEVIER SCIENCE PUBLISHERS BV., BARKING, GB, vol. 21, no. 4, February 2000 (2000-02), pages 345-351, XP004184116 ISSN: 0142-9612

## Description

### TECHNICAL FIELD

In one aspect, the present invention relates to a process of treating implantable medical devices with coating compositions to provide the release of bioactive (e.g., pharmaceutical) agents from the surface of the devices under physiological conditions. In another aspect, the invention relates to the coating compositions, *per se,* and to devices or surfaces coated with such compositions. In yet another aspect, the invention relates to methods of coating compositions on devices.

### BACKGROUND OF THE INVENTION

Many surgical interventions require the placement of a medical device into the body. While necessary and beneficial for treating a variety of medical conditions, the placement of metal or polymeric devices in the body gives rise to numerous complications. Some of these complications include: increased risk of infection; initiation of a foreign body response resulting in inflammation and fibrous encapsulation; and initiation of a wound healing response resulting in hyperplasia and restenosis. These, and other complications must be dealt with when introducing a metal or polymeric device into the body.

One approach to reducing the potential harmful effects of such an introduction is to attempt to provide a more biocompatible implantable device. While there are several methods available to improve the biocompatibility of implantable devices, one method which has met with limited success is to provide the device with the ability to deliver bioactive compounds to the vicinity of the implant. By so doing, some of the harmful effects associated with the implantation of medical devices can be diminished. Thus, for example, antibiotics can be released from the surface of the device to minimize the possibility of infection, and anti-proliferative drugs can be released to inhibit hyperplasia. Another benefit to the local release of bioactive agents is the avoidance of toxic concentrations of drugs which are sometimes necessary, when given systemically, to achieve therapeutic concentrations at the site where they are needed.

Although the potential benefits expected from the use of medical devices capable of releasing pharmaceutical agents from their surfaces is great, the development of such medical devices has been slow. This development has been hampered by the many challenges that need to be successfully overcome when undertaking said development. Some of these challenges are: 1) the requirement, in some instances, for long term release of bioactive agents; 2) the need for a biocompatible, non-inflammatory device surface; 3) the need for significant durability, particularly with devices that undergo flexion and/or expansion when being implanted or used in the body; 4) concerns regarding processability, to enable the device to be manufactured in an economically viable and reproducible manner; and 5) the requirement that the finished device be sterilizable using conventional methods.

Several implantable medical devices capable of delivering medicinal agents have been described. Several patents are directed to devices utilizing biodegradable or bioresorbable polymers as drug containing and releasing coatings, including Tang *et al* , U.S. Patent 4,916,193 and MacGregor, U.S. Patent 4,994,071. Other patents are directed to the formation of a drug containing hydrogel on the surface of an implantable medical device, these include Amiden *et al,* U.S. patent 5,221,698 and Sahatjian, U.S. Patent 5,304,121. Still other patents describe methods for preparing coated intravascular stents via application of polymer solutions containing dispersed therapeutic material to the stent surface followed by evaporation of the solvent. This method is described in Berg *et al*, U.S. Patent 5,464,650.

However, there remain significant problems to be overcome in order to provide a therapeutically significant amount of a bioactive compound on the surface of the implantable medical device. This is particularly true when the coated composition must be kept on the device in the course of flexion and/or expansion of the device during implantation or use. It is also desirable to have a facile and easily processable method of controlling the rate of bioactive release from the surface of the device.

Although a variety of hydrophobic polymers have previously been described for use as drug release coatings, Applicant has found that only a small number possess the physical characteristics that would render them useful for implantable medical devices which undergo flexion and/or expansion upon implantation. Many polymers which demonstrate good drug release characteristics, when used alone as drug delivery vehicles, provide coatings that are too brittle to be used on devices which undergo flexion and/or expansion. Other polymers can provoke an inflammatory response when implanted. These or other polymers demonstrate good drug release characteristics for one drug but very poor characteristics for another.

Some polymers show good durability and flexibility characteristics when applied to devices without drug, but lose these favorable characteristics when drug is added. Furthermore, often times the higher the concentration of drugs or the thicker the application of polymer to the device surface, the poorer the physical characteristics of the polymer become. It has been very difficult to identify a polymer which provides the proper physical characteristics in the presence of drugs and one in which the drug delivery rate can be controlled by altering the concentration of the drug in the polymer or the thickness of the polymer layer.

Applicants have previously provided an implantable medical device that can undergo flexion and/or expansion upon implantation, and that is also capable of delivering a therapeutically significant amount of a pharmaceutical agent or agents from the surface of the device. Applicant's issued US Patent No. 6,214,901 and published PCT Application No. WO 00/55396 provide a coating composition that comprises at least one polyalkyl(meth)acrylate, as a first polymeric component and poly(ethylene-co-vinyl acetate) ("pEVA") as a second polymeric component, and describe the use of such compositions for coating an implant surface using any suitable means, e.g., by dipping, spraying and the like.

While certainly suitable for their intended use, Applicants have found that devices coated with such compositions have the potential to exhibit properties with detectable, and undesirable, variability, for instance, when evaluated using an "accelerated bioactive release" test method, or a "bioactive agent elution" test method, as described herein. It would be helpful to find ways of affecting, and preferably controlling, the potential for such variability, in order to provide coated devices with uniform properties.

Various other references relate to the use of coatings to provide implantable medical devices with bioactive agents. See, for instance, US 20020007213, and published PCT Application Nos. WO 200187372, WO 200187373, WO 200187374, WO 200187375, WO 200187376, WO 200226139, WO 200226271, WO 200226281, WO 200187342, and WO 200187263

Finally, Applicant's corresponding US application, filed on a date even herewith and having Attorney Docket No. 9896.129.9, describes a coating composition having first and second polymer components and bioactive agent, and a related method of coating a device that includes, in preferred embodiment, the step of applying the composition (including components or layers thereof) to the device surface under conditions of controlled humidity (at a given temperature), for instance, under conditions of increased or decreased relative humidity (as compared to ambient humidity).

### BRIEF DESCRIPTION OF THE DRAWING

In the Drawing:
Figure 1 provides a plot showing the experimental results described in Example 1.
Figure 2 provides a plot showing the experimental results described in Example 2.
Figure 3 provides a plot showing the experimental results described in Example 3.

### SUMMARY OF THE INVENTION

The term "coating composition", as used herein, will refer to one or more vehicles (e.g., a system of solutions, mixtures, emulsions, dispersions, blends etc.) used to effectively coat a surface with bioactive agent, first polymer component and/or second polymer component, either individually or in any suitable combination. In turn, the term "coated composition" will refer to the effective combination, upon a surface, of bioactive agent, first polymer component and second polymer component, whether formed as the result of one or more coating vehicles, or in one or more layers.

The present invention provides a coating composition, and related method for using the coating composition to coat a surface with a bioactive agent, for instance to coat the surface of an implantable medical device in a manner that permits the surface to release the bioactive agent over time when implanted *in vivo.*

The coating composition comprises a bioactive agent in combination with a plurality of polymers, including a first polymer component comprising at least one aromatic poly(meth)acrylate and a second polymer component comprising poly(ethylene-co-vinyl acetate). In a further preferred embodiment, the device is a medical device that undergoes flexion and/or expansion in the course of implantation or use *in vivo.*

Applicants have discovered the manner in which the inclusion of one or more aromatic poly(meth)acrylates as the first polymeric component, permit the use of a broad array of bioactive agents, particularly in view of the use of a corresponding broad array of solvents. For instance, such compositions of this invention permit the inclusion of polar bioactive agents, by the use of solvents and solvent systems that are themselves more polar than typically used.

In one such an embodiment, the composition preferably comprises at least one first polymeric component selected from the group consisting of polyaryl(meth)acrylates, polyaralkyl(meth)acrylates, and polyaryloxyalkyl(meth)acrylates. Such terms are used to describe polymeric structures wherein at least one carbon chain and at least one aromatic ring are combined with acrylic groups, typically esters, to provide a composition of this invention. For instance, and more specifically, a polyaralkyl(meth)acrylate or polyarylalky(meth)acrylate can be made from aromatic esters derived from alcohols also containing aromatic moieties.

In a further preferred embodiment, the method of coating a device comprises the step of applying the composition (including layers or components thereof) to the device surface under conditions of controlled relative humidity (at a given temperature), for instance, under conditions of increased or decreased relative humidity as compared to ambient humidity.

Humidity can be "controlled" in any suitable manner, including at the time of preparing and/or using (as by applying) the composition (for instance, by coating the surface in a confined chamber or area adapted to provide a relative humidity different than ambient conditions), and/or by adjusting the water content of the coating or coated composition itself. In turn, even ambient humidity can be considered "controlled" humidity for purposes of this invention, if indeed it has been correlated with and determined to provide a corresponding controlled bioactive release profile.

Moreover, and particularly when coating a plurality of coating compositions (including components thereof) in the form of a corresponding plurality of layers, humidity can be controlled in different ways (e.g., using a controlled environment as compared to a hydrated or dehydrated coating composition) and/or at different levels to provide a desired release profile for the resulting composite composition coating. As described and exemplified below, a resultant composition can be coated using a plurality of individual steps or layers, including for instance, an initial layer having only bioactive agent (or bioactive agent with one or both of the polymeric components), over which are coated one or more additional layers containing suitable combinations of bioactive agent, first polymeric component and/or second polymeric component, the combined result of which is to provide a coated composition of the invention. In turn, and in a particularly preferred embodiment, the invention further provides a method of reproducibly controlling the release (e.g., elution) of a bioactive agent from the surface of a medical device implanted *in vivo,* the method comprising the step of coating the device with a coating composition comprising the bioactive agent under conditions of controlled humidity. Applicants have discovered that coating compositions of this invention under conditions of increased humidity will typically accelerate release of the bioactive agent *in vivo,* while decreasing humidity levels will tend to decelerate release. The controlled humidity can be accomplished by any suitable means, e.g., by controlling humidity in the environment during the coating process and/or by hydrating the coating composition itself

Moreover, a plurality of coating compositions and corresponding coating steps can be employed, each with its own controlled humidity, in order to provide a desired combination of layers, each with its corresponding release profile. Those skilled in the art will appreciate the manner in which the combined effect of these various layers can be used and optimized to achieve various effects *in vivo.*

While not intending to be bound by theory, the release kinetics of the bioactive agent *in vivo* are thought to generally include both a short term ("burst") release component, within the order of minutes to hours or less after implantation, and a longer term release component, which can range from on the order of hours to days or even months of useful release. As used herein, the "acceleration" or "deceleration" of bioactive release can include either or both of these release kinetics components.

In yet another embodiment, the present invention comprises a method for selecting an optimal release rate from a coated composition, the method comprising the steps of coating sample surfaces at a plurality of different humidity levels and evaluating the corresponding release profiles to determine a controlled humidity level corresponding to a desired profile. In a related embodiment, the invention provides a chamber for use in coating a medical device with a coating composition of the present invention under conditions of controlled humidity.

In one such embodiment, for instance, the coating composition is coated onto the device under relative humidity controlled at a level of between about 0% and about 95 % relative humidity (at a given temperature, between about 15°C and 30°C), and more preferably between about 0% and about 50% relative humidity. Without intending to be bound by theory, Applicants have found that potential differences in the ambient humidity, as between coating runs at the same location, and/or as between different coating locations, can vary significantly, and in a manner that might affect such properties as the release or elution of the bioactive agent. By using a controlled humidity, Applicants can provide a coating in a manner that is significantly more controllable and reproducible.

Additionally, the ability to coat a device in the manner of the present invention provides greater latitude in the composition of various coating layers, e.g., permitting more or less of the aromatic poly(meth)acrylate (and/or polyalkyl(meth)acrylate, if present) to be used in coating compositions used to form different layers (e.g., as a topcoat layer). This, in turn, provides the opportunity to further control release and elution of the bioactive agent from the overall coating.

A coating composition can be provided in any suitable form, e.g., in the form of a true solution, or fluid or paste-like emulsion, mixture, dispersion or blend. In turn, the coated composition will generally result from the removal of solvents or other volatile components and/or other physical-chemical actions (e.g., heating or illuminating) affecting the coated composition *in situ* upon the surface.

In a further embodiment the coated composition can further comprise at least one polyalkyl(meth)acrylate, as a first polymeric component, and poly(ethylene-co-vinyl acetate) ("pEVA") as a second polymeric component. A particularly preferred polymer mixture for use in this invention includes mixtures of poly(n-butyl methacrylate) ("pBMA") and poly(ethylene-*co*-vinyl acetate) co-polymers (pEVA). This mixture of polymers has proven useful with absolute polymer concentrations (i.e., the total combined concentrations of both polymers in the coating composition), of between about 0.05 and about 70 percent (by weight of the coating composition). In a further embodiment the polymer mixture includes a polyalkyl(meth)acrylate (such as poly(n-butyl methacrylate)) with a weight average molecular weight of from about 100 kilodaltons to about 1000 kilodaltons and a pEVA copolymer with a vinyl acetate content of from about 20 to about 40 weight percent.

In a further embodiment the polymer mixture includes a polyalkyl(meth)acrylate (e.g., poly(n-butyl methacrylate)) with a molecular weight of from about 200 kilodaltons to about 500 kilodaltons and a pEVA copolymer with a vinyl acetate content of from about 30 to about 34 weight percent. The concentration of the bioactive agent or agents dissolved or suspended in the coating mixture can range from about 0.01 to about 90 percent, by weight, based on the weight of the final coating composition.

Coating compositions comprising aromatic poly(meth)acrylates provide unexpected advantages in certain applications, even as compared to coating compositions that instead employ only polyalkyl(meth)acrylates. Such advantages relate, for instance, to the ability to provide coatings with different characteristics (e.g., different solubility characteristics) than other coatings (e.g., those that include a polyalkyl(meth)acrylate component), while maintaining an optimal combination of other desired properties. Without intending to be bound by theory, it would appear that the increased solubility (particularly in more polar solvents) that is provided by an aromatic, rather than alkyl poly(meth)acrylate of this invention, permits the use of poly(ethylene-co-vinyl acetate) components that are themselves more polar (e.g., having significantly greater vinyl acetate concentrations) than those typically preferred for use with the polyalkyl(meth)acrylates.

Suitable polymers, and bioactive agents, for use in preparing coating compositions of the present invention can be prepared using conventional organic synthetic procedures and/or are commercially available from a variety of sources, including for instance, from Sigma Aldrich (e.g., 1,3-dioxolane, vincristine sulfate, and poly(ethylene-co-vinylacetate), and Polysciences, Inc, Warrington, PA (e.g., polybenzylmethacryate and poly(methyl methacrylate-co-n-butyl methacrylate). Optionally, and preferably, such polymer components are either provided in a form suitable for *in vivo* use, or are purified for such use to a desired extent (e.g., by removing impurities) by conventional methods available to those skilled in the art.

The coating composition and method can be used to control the amount and rate of bioactive agent (e.g., drug) release from one or more surfaces of implantable medical devices. In a preferred embodiment, the method employs a mixture of hydrophobic polymers in combination with one or more bioactive agents, such as a pharmaceutical agent, such that the amount and rate of release of agent(s) from the medical device can be controlled, e.g., by adjusting the relative types and/or concentrations of hydrophobic polymers in the mixture. For a given combination of polymers, for instance, this approach permits the release rate to be adjusted and controlled by simply adjusting the relative concentrations of the polymers in the coating mixture.

A preferred coating composition of this invention includes a mixture of two or more polymers having complementary physical characteristics, and a pharmaceutical agent or agents applied to the surface of an implantable medical device which undergoes flexion and/or expansion upon implantation or use. The applied coating composition is cured (e.g., solvent evaporated) to provide a tenacious and flexible bioactive-releasing coated composition on the surface of the medical device. The complementary polymers are selected such that a broad range of relative polymer concentrations can be used without detrimentally affecting the desirable physical characteristics of the polymers. By use of the polymer mixtures of the invention the bioactive release rate from a coated medical device can be manipulated by adjusting the relative concentrations of the polymers.

### DETAILED DESCRIPTION OF THE INVENTION

In a particularly preferred embodiment, the present invention relates to a coating composition and related method for coating an implantable medical device which undergoes flexion and/or expansion upon implantation. The structure and composition of the underlying device can be of any suitable, and medically acceptable, design and can be made of any suitable material that is compatible with the coating itself. The surface of the medical device is provided with a coating containing one or more bioactive agents.

In order to provide a preferred coating, a coating composition is prepared to include a solvent, a combination of complementary polymers dissolved in the solvent, and the bioactive agent or agents dispersed in the polymer/solvent mixture. The solvent is preferably one in which the polymers form a true solution. The pharmaceutical agent itself may either be soluble in the solvent or form a dispersion throughout the solvent. For instance, Applicant's previous US Patent No. 6,214,901 exemplifies the use of tetrahydrofuran as a solvent. While THF is certainly suitable, and at times is preferred, for certain coating compositions, Applicants have further discovered that other solvents can be used as well, in order to provide unexpected advantages. These solvents include, but are not limited to, alcohols (e.g., methanol, butanol, propanol and isopropanol), alkanes (e.g., halogenated or unhalogenated alkanes such as hexane and cyclohexane), amides (e.g., dimethylformamide), ethers (e.g., THF and dioxolane), ketones (e.g., methylethylketone), aromatic compounds (e.g., toluene and xylene), nitriles (e.g., acetonitrile) nd esters (e.g., ethyl acetate).

The resultant coating composition can be applied to the device in any suitable fashion, under conditions of controlled relative humidity, e.g., it can be applied directly to the surface of the medical device, or alternatively, to the surface of a surface-modified medical device, by dipping, spraying, or any conventional technique. In one such embodiment, for instance, the coating comprises at least two layers, which are either coated under different conditions of relative humidity and/or which are themselves different. For instance, a base layer having either bioactive agent alone, or together with one or more of the polymeric components, after which one or more topcoat layers are coated, each with or without bioactive agent and/or each under different conditions of relative humidity. These different layers, in turn, can cooperate in the resultant composite coating to provide an overall release profile having certain desired characteristics, and is particularly preferred for use with bioactive agents of high molecular weight. Preferably, the composition is coated onto the device surface in one or more applications. The method of applying the coating composition to the device is typically governed by the geometry of the device and other process considerations. The coating is subsequently cured by evaporation of the solvent. The curing process can be performed at room temperature, elevated temperature, or with the assistance of vacuum.

The polymer mixture for use in this invention is preferably biocompatible, e.g., such that it results in no induction of inflammation or irritation when implanted. In addition, the polymer combination must be useful under a broad spectrum of both absolute concentrations and relative concentrations of the polymers. This means that the physical characteristics of the coating, such as tenacity, durability, flexibility and expandability, will typically be adequate over a broad range of polymer concentrations. Furthermore, the ability of the coating to control the release rates of a variety of pharmaceutical agents can preferably be manipulated by varying the absolute and relative concentrations of the polymers.

A first polymer component of this invention provides an optimal combination of various structural/functional properties, including hydrophobicity, durability, bioactive agent release characteristics, biocompatibility, molecular weight, and availability.

Suitable first polymers are aromatic poly(meth)acrylates including polyaryl(meth)acrylates, polyaralkyl(meth)acrylates, and polyaryloxyalkyl(meth)acrylates, in particular those with aryl groups having from 6 to 16 carbon atoms and with weight average molecular weights from about 50 to about 900 kilodaltons. Examples of polyaryl(meth)acrylates include poly-9-anthracenylmethacrylate, polychlorophenylacrylate, polymethacryloxy-2-hydroxybenzophenone, polymethacryloxybenzotriazole, polynaphthylacrylate, polynaphthylmethacrylate, poly-4-nitrophenylacrylate, polypentachloro(bromo, fluoro)acrylate and methacrylate, polyphenylacrylate and methacrylate. Examples of polyaralkyl(meth)acrylates include polybenzylacrylate and methacrylate, poly-2-phenethylacrylate and methacrylate, poly-1-pyrenylmethylmethacrylate. Examples of polyaryloxyalkyl(meth)acrylates include polyphenoxyethylacrylate and methacrylate, polyethyleneglycolphenylether acrylates and methacrylates with varying polyethyleneglycol molecular weights.

A second polymer component of this invention provides an optimal combination of similar properties, and particularly when used in admixture with the first polymer component. Examples of suitable second polymers are available commercially and include poly(ethylene-co-vinyl acetate) having vinyl acetate concentrations of between about 8% and about 90%, in the form of beads, pellets, granules, etc. pEVA co-polymers with lower percent vinyl acetate become increasingly insoluble in typical solvents.

A particularly preferred coating composition for use in this invention includes mixtures of aromatic poly(meth)acrylates (e.g., polybenzyl(meth)acrylate) and poly(ethylene-co-vinyl acetate) co-polymers (pEVA). This mixture of polymers has proven useful with absolute polymer concentrations (i.e., the total combined concentrations of both polymers in the coating composition), of between about 0.05 and about 70 percent (by weight), and more preferably between about 0.25 and about 10 percent (by weight). In one preferred embodiment the polymer mixture includes a first polymeric component with a weight average molecular weight of from about 100 kilodaltons to about 500 kilodaltons and a pEVA copolymer with a vinyl acetate content of from about 8 to about 90 weight percent, and more preferably between about 20 to about 40 weight percent. In a particularly preferred embodiment the polymer mixture includes a first polymeric component with a molecular weight of from about 200 kilodaltons to about 400 kilodaltons and a pEVA copolymer with a vinyl acetate content of from about 30 to about 34 weight percent. The concentration of the bioactive agent or agents dissolved or suspended in the coating mixture can range from about 0.01 to about 90 percent, by weight, based on the weight of the final coating composition.

The bioactive (e.g., pharmaceutical) agents useful in the present invention include virtually any therapeutic substance which possesses desirable therapeutic characteristics for application to the implant site. These agents include: thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives (including antiangiogenesis agents), anticancer chemotherapeutic agents, anti-inflammatory steroid or non-steroidal anti-inflammatory agents, immunosuppressive agents, growth hormone antagonists, growth factors, dopamine agonists, radiotherapeutic agents, peptides, proteins, enzymes, extracellular matrix components, ACE inhibitors, free radical scavengers, chelators, antioxidants, anti polymerases, antiviral agents, photodynamic therapy agents, and gene therapy agents.

A coating composition of this invention can be used to coat the surface of a variety of devices, and is particularly useful for those devices that will come in contact with aqueous systems. Such devices are coated with a composition adapted to release bioactive agent in a prolonged and controlled manner, generally beginning with the initial contact between the device surface and its aqueous environment.

A coating composition of this invention is preferably used to coat an implantable medical device that undergoes flexion or expansion in the course of its implantation or use in vivo. The words "flexion" and "expansion" as used herein with regard to implantable devices will refer to a device, or portion thereof, that is bent (e.g., by at least 45 degrees or more) and/or expanded (e.g., to more than twice its initial dimension), either in the course of its placement, or thereafter in the course of its use *in vivo.*

Examples of suitable catheters include urinary catheters, which would benefit from the incorporation of antimicrobial agents (e.g., antibiotics such as vancomycin or norfloxacin) into a surface coating, and intravenous catheters which would benefit from antimicrobial agents and or from antithrombotic agents (e.g., heparin, hirudin, coumadin). Such catheters are typically fabricated from such materials as silicone rubber, polyurethane, latex and polyvinylchloride.

The coating composition can also be used to coat stents, e.g., either self-expanding stents, which are typically prepared from nitinol, or balloon-expandable stents, which are typically prepared from stainless steel. Other stent materials, such as cobalt chromium alloys, can be coated by the coating composition as well.

A coating composition of the present invention can be used to coat an implant surface using any suitable means, e.g., by dipping, spraying and the like. The suitability of the coating composition for use on a particular material, and in turn, the suitability of the coated composition can be evaluated by those skilled in the art, given the present description.

The overall weight of the coating upon the surface is typically not critical. The weight of the coating attributable to the bioactive agent is preferably in the range of about one microgram to about 10 mg of bioactive agent per cm² of the effective surface area of the device. By "effective" surface area it is meant the surface amenable to being coated with the composition itself. For a flat, nonporous, surface, for instance, this will generally be the macroscopic surface area itself, while for considerably more porous or convoluted (e.g., corrugated, pleated, or fibrous) surfaces the effective surface area can be significantly greater than the corresponding macroscopic surface area. More preferably, the weight of the coating attributable to the bioactive is between about 0.01 mg and about 0.5 mg of bioactive agent per cm² of the gross surface area of the device. This quantity of drug is generally required to provide adequate activity under physiological conditions.

In turn, the final coating thickness of a presently preferred coated composition will typically be in the range of about 0.1 micrometers to about 100 micrometers, and preferably between about 0.5 micrometers and about 25 micrometers. This level of coating thickness is generally required to provide an adequate concentration of drug to provide adequate activity under physiological conditions.

The coated composition provides a means to deliver bioactive agents from a variety of biomaterial surfaces. Preferred biomaterials include those formed of synthetic polymers, including oligomers, homopolymers, and copolymers resulting from either addition or condensation polymerizations. Examples of suitable addition polymers include, but are not limited to, acrylics such as those polymerized from methyl acrylate, methyl methacrylate, hydroxyethyl methacrylate, hydroxyethyl acrylate, acrylic acid, methacrylic acid, glyceryl acrylate, glyceryl methacrylate, methacrylamide, and acrylamide; vinyls such as ethylene, propylene, styrene, vinyl chloride, vinyl acetate, vinyl pyrrolidone, and vinylidene difluoride. Examples of condensation polymers include, but are not limited to, nylons such as polycaprolactam, polylauryl lactam, polyhexamethylene adipamide, and polyhexamethylene dodecanediamide, and also polyurethanes, polycarbonates, polyamides, polysulfones, poly(ethylene terephthalate), polylactic acid, polyglycolic acid, polydimethylsiloxanes, and polyetheretherketone.

Certain natural materials are also suitable biomaterials, including human tissue such as bone, cartilage, skin and teeth; and other organic materials such as wood, cellulose, compressed carbon, and rubber. Other suitable biomaterials include metals and ceramics. The metals include, but are not limited to, titanium, stainless steel, and cobalt chromium. A second class of metals include the noble metals such as gold, silver, copper, and platinum. Alloys of metals may be suitable for biomaterials as well. The ceramics include, but are not limited to, silicon nitride, silicon carbide, zirconia, and alumina, as well as glass, silica, and sapphire. Combinations of ceramics and metals would be another class of biomaterials. Another class of biomaterials are fibrous or porous in nature. The surface of such biomaterials can be pretreated (e.g., with a Parylene coating composition) in order to alter the surface properties of the biomaterial.

Biomaterials can be used to fabricate a variety of implantable devices. General classes of suitable implantable devices include, but are not limited to, vascular devices such as grafts, stents, catheters, valves, artificial hearts, and heart assist devices; orthopedic devices such as joint implants, fracture repair devices, and artificial tendons; dental devices such as dental implants and fracture repair devices; drug delivery devices; ophthalmic devices and glaucoma drain shunts; urological devices such as penile, sphincter, urethral, bladder, and renal devices; and other catheters, synthetic prostheses such as breast prostheses and artificial organs. Other suitable biomedical devices include dialysis tubing and membranes, blood oxygenator tubing and membranes, blood bags, sutures, membranes, cell culture devices, chromatographic support materials, biosensors, and the like.

The invention will be further described with reference to the following nonlimiting Examples. It will be apparent to those skilled in the art that many changes can be made in the embodiments described without departing from the scope of the present invention. Thus the scope of the present invention should not be limited to the embodiments described in this application, but only by the embodiments described by the language of the claims and the equivalents of those embodiments. Unless otherwise indicated, all percentages are by weight.

### EXAMPLES

### Test Methods

The potential suitability of particular coated compositions for in vivo use can be determined by a variety of methods, including the Durability, Flexibility and Release Tests, examples of each of which are described herein.

### Sample Preparation

One millimeter diameter stainless steel wires (e.g. 304 grade) are cut into 5 centimeter lengths. The wire segments can be treated with a Parylene C coating composition (Parylene is a trademark of the Union Carbide Corporation) or evaluated with no treatment. The wire segments are weighed on a micro-balance.

Bioactive agent/polymer mixtures are prepared at a range of concentrations in an appropriate solvent, in the manner described herein. The coating mixtures are applied to respective wires, or portions thereof, by dipping or spraying, and the coated wires are allowed to cure by solvent evaporation. The coated wires are re-weighed. From this weight, the mass of the coating is calculated, which in turn permits the mass of the coated polymer(s) and bioactive agent to be determined. The coating thickness can be measured using any suitable means, e.g., by the use of a microprocessor coating thickness gauge (Minitest 4100).

The Durability and Flexibility of the coated composition can be determined in the following manner.

### Durability Test

A suitable Durability Test, involves a method in which a coated specimen (e.g., wire) is subjected to repeated frictional forces intended to simulate the type of abrasion the sample would be exposed to in actual use.

The Test described below employs a repetitive 60 cycle treatment, and is used to determine whether there is any change in force measurements between the first 5 cycles and the last 5 cycles, or whether there is any observable flaking or scarring detectable by scanning electron microscopy ("SEM") analysis. Regenerated cellulose membrane is hydrated and wrapped around a 200 gram stainless steel sled. The cellulose membrane is clipped tightly on the opposite side of the sled. The sled with rotatable arm is then attached to a 250 gram digital force gauge with computer interface. The testing surface is mounted on a rail table with micro-stepper motor control. The wires are clamped onto the test surface. The cellulose covered sled is placed on top of the wires. Initial force measurements are taken as the sled moves at 0.5 cm/sec over a 5 cm section for 5 push/pull cycles. The sled then continues cycling over the coated samples for 50 push/pull cycles at 5 cm/sec to simulate abrasion. The velocity is then reduced to 0.5 cm/sec and the final force measurements are taken over another 5 push/pull cycles.

SEM micrographs are taken of abraded and nonabraded coated wires to evaluate the effects of the abrasion on the coating.

### Flexibility Test

A suitable Flexibility Test, in turn, can be used to detect imperfections (when examined by scanning electron microscopy) that develop in the course of flexing of a coated specimen, and in particular, signs of cracking at or near the area of a bend.

A wire specimen is obtained and coated in the manner described above. One end of the coated wire (1.0 cm) is clamped in a bench vice. The free end of the wire (1.0 cm) is held with a pliers. The wire is bent until the angle it forms with itself is less than 90 degrees. The wire is removed from the vice and examined by SEM to determine the effect of the bending on the coating.

### Bioactive Agent Release Assay

A suitable Bioactive Agent Release Assay, as described herein, can be used to determine the extent and rate of drug release under physiological conditions. In general it is desirable that less than 50% of the total quantity of the drug released, be released in the first 24 hours. It is frequently desirable for quantities of drug to be released for a duration of at least 30 days. After all the drug has been released, SEM evaluation should reveal an intact coating.

Except as otherwise provided herein, each coated wire is placed in a test tube with 5 ml of buffer, which unless stated otherwise herein, was provided in the form of Phosphate Buffered Saline ("PBS", 10 mM phosphate, 150 mM NaCl, pH 7.4, aqueous solution).

The tubes are placed on a rack in an environmental orbital shaker and agitated at 37°C. At timed intervals, the PBS is removed from the tube and replaced with fresh PBS. The drug concentration in each PBS sample is determined using the appropriate method.

After all measurable drug has been released from the coated wire, the wire is washed with water, dried, and re-weighed, the coating thickness re-measured, and the coating quality examined by SEM analysis.

### Reference Example 1

### Release of Hexachlorophene from Coated Stainless Steel Wires

A one millimeter diameter stainless steel wire (304 grade) was cut into two centimeter segments. The segments were treated with a Parylene C coating composition in order to deposit a thin, conformal, polymeric coating on the wires.

Four solutions were prepared for use in coating the wires. The solutions included mixtures of: pEVA (33 weight percent vinyl acetate, from Aldrich Chemical Company, Inc.); poly(n-butyl methacrylate "pBMA") (337,000 average molecular weight, from Aldrich Chemical Company, Inc.); and hexachlorophene ("HCP") from Sigma Chemical Co., dissolved in tetrahydrofuran. The solutions were prepared as follows:
1) 10 mg/ml pEVA//60mg/ml pBMA//100mg/ml HCP
2) 35 mg/ml pEVA//35mg/ml pBMA//100mg/ml HCP
3) 60 mg/ml pEVA//10mg/ml pBMA//100mg/ml HCP
4) 0 mg/ml pEVA//0mg/ml pBMA//100mg/ml HCP

Nine wire segments were coated with each coating solution. The following protocol was followed for coating the wire segments. The Parylene-treated wire segments were wiped with an isopropyl alcohol dampened tissue prior to coating. The wire segments were dipped into the coating solution using a 2cm/second dip speed. The wire segments were immediately withdrawn from the coating solution at a rate of 1 cm/second, after which the coated segments were air-dried at room temperature.

Individual wire segments were placed in tubes containing 2 ml of phosphate buffered saline ("PBS", pH 7.4). The tubes were incubated at 37 degrees centigrade on an environmental, orbital shaker at 100 rotations/minute. The PBS was changed at 1 hour, 3 hours, and 5 hours on the first day, and daily thereafter. The PBS samples were analyzed for HCP concentration by measuring the absorbance of the samples at 298 nms on a UV/visible light spectrophotometer and comparing to an HCP standard curve.

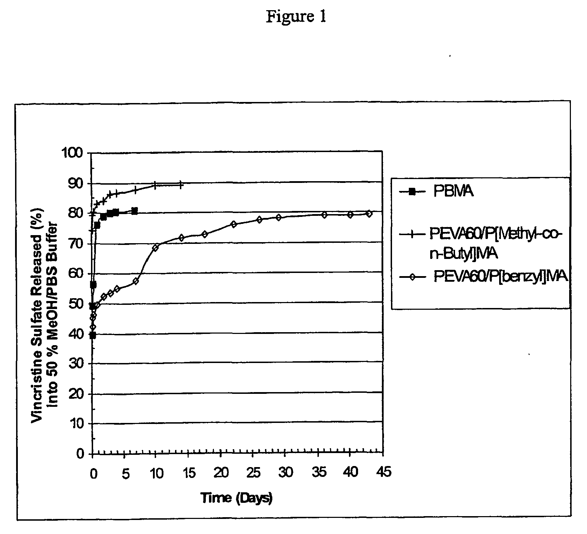

Results are provided in Figure 1 of US Patent 6,214,901, which demonstrates the ability to control the release rate of a pharmaceutical agent from a coated surface by varying the relative concentrations of a polymer mixture.

### Reference Example 2

The polymers described in this disclosure have been evaluated using an Assay protocol as outlined above. The polymer mixtures evaluated have ranged from 100% pBMA to 100% pEVA. Representative results of those evaluations are summarized below.

Control coatings that are made up entirely of pBMA are very durable showing no signs of wear in the Durability Test. When subjected to the Flexibility Test, however, these coatings develop cracks, particularly in the presence of significant concentrations of drug. These coatings also release drug very slowly.

Control coatings that are made up entirely of pEVA, in contrast, are less durable and show no signs of cracking in the Flexibility Test, but develop significant scarring in the Durability Test. These coatings release drugs relatively rapidly, usually releasing more than 50% of the total within 24 hours.

These coatings , which contain a mixture of both polymers, are very durable, with no signs of wear in the Durability Test and no cracking in the Flexibility Test. Drug release from these coatings can be manipulated by varying the relative concentrations of the polymers. For instance, the rate of drug release can be controllably increased by increasing the relative concentration of pEVA.

Bioactive agent containing coatings which show no signs of scarring in the Durability Test and no cracking in the Flexibility Test possess the characteristics necessary for application to implantable medical devices that undergo flexion and/or expansion in the course of implantation and/or use.

### Comparative EXAMPLE 1

Three different polymer solutions, each at a concentration of 35 mg/ml, were prepared in 1,3-dioxolane in the manner provided below in order to provide coating compositions in the form of one part systems. The first solution contained poly(n-butyl methacrylate), with approximate weight average molecular weight of 337 kilodaltons; the second solution contained poly(ethylene-co-vinylacetate), with a vinyl acetate content of 60 % (w/w) and poly(benzyl methacrylate) ("PEVA60/P[benzyl]MA"), in a polymer ratio of (50/50 % w/w), respectively. The poly(n-butyl methacrylate) and poly(ethylene-co-vinylacetate) were purified by extraction with organic solvents to remove impurities, e.g., monomer residues. The third solution contained poly(ethylene-co-vinylacetate) with a vinyl acetate content of 60 % (w/w) and poly(methyl methacrylate-co-n-butyl methacrylate) ("PEVA60/P[Methyl-co-n-Butyl]MA"), commercially available and known as poly(methyl methacrylate/n-butyl methacrylate), in a polymer ratio of (50/50 % w/w), respectively. Vincristine sulfate and some additional 1,3-dioxolane were added to each of the three solutions in order to provide coating compositions in the form of one part systems (at final concentrations of 17.5 mg/ml). The vincristine/polymer ratio was 30/70 % (w/w).

### Sample preparation

Sixteen-millimeter diameter stainless steel discs with an overall thickness of two millimeters were fabricated with a fourteen-millimeter diameter flat pedestal. The pedestal had a surface area of 1.54 cm². A surface treatment such as Parylene could be applied to the disc or the surface could be left untreated. The discs were weighed on a microbalance.

Polymer solutions containing vincristine sulfate were applied to the pedestal surface of the discs with a pipette. Two coats were applied with drying of the coat between applications. The solvent was evaporated from the discs and the discs were re-weighed on a microbalance to obtain the amount of vincristine sulfate per disc.

### Bioactive Agent Release Assay

A suitable Bioactive Agent Release Assay, as described herein, can be used to determine the extent and rate of bioactive agent release. In general it is desirable that less than 50 % of the total quantity of the bioactive agent be released in the first 24 hours. It is frequently desirable for quantities of bioactive agent to be released for a duration of at least 30 days.

Except as otherwise provided herein, each coated disc was placed in an amber vial with 4 mls of elution solvent. The elution solvent was composed of 50 % methanol and 50 % PBS. The vials were placed in a water bath and stirred at 37 °C. At time intervals, the disc was removed from the vial, placed into a new vial containing fresh elution solvent and the new vial was placed into the water bath to continue the experiment. The bioactive agent concentration in each elution solvent sample was determined using UV spectroscopy.

After all measurable bioactive agent was released from the coated disc; the disc was washed with water, dried and re-weighed to determine the weight loss of the disc.

### Conclusions

Results are provided in Figure 1, where it can be seen that approximately 80 % or more of the vincristine sulfate was released within 1 day for coatings that contained either poly(n-butyl methacrylate) or a blend of poly(methyl methacrylate-co-n-butyl methacrylate) and poly(ethylene-co-vinylacetate). The blend containing poly(benzyl methacrylate) and poly(ethylene-co-vinylacetate) showed sustained controlled release of vincristine sulfate for more than a one-month period.

### HUMIDITY EXAMPLES

Two examples are provided using two different bioactive agents, namely, β-estradiol, as an example of a low molecular weight bioactive agent that weighs 272 daltons, and tetramethylrhodamine isothiocyanate-Dextran (dextran-TRITC) as an example of a water soluble, high molecular weight bioactive agent that weighs 4400 daltons.

### Reference EXAMPLE 2

### Solution preparation - dextran

Two solutions were prepared in order to provide a coating composition of Applicant's copending application in the form of a two part system. The first solution was an aqueous solution containing the bioactive agent, dextran-TRITC, at a concentration of 15 mg/ml. The second solution contained poly(ethylene-co-vinylacetate) with a vinyl acetate concentration of 33 % (w/w) and poly(n-butyl methacrylate), with approximate weight average molecular weight of 337 kilodaltons. The poly(n-butyl methacrylate) and poly(ethylene-co-vinylacetate) were purified by extraction with organic solvents to remove impurities, e.g., monomer residues. The polymers of the second solution were dissolved in tetrahydrofuran at a concentration of 10 mg/ml.

### Sample preparation

Fifteen-millimeter diameter stainless steel discs with an overall thickness of two millimeters were fabricated with a nine-millimeter diameter flat pedestal. The pedestal had a surface area of 0.64 cm². A surface treatment such as Parylene could be applied to the disc or the surface could be left untreated. The discs were weighed on a microbalance. The aqueous bioactive agent solution was applied to the pedestal surface of the discs with a pipette. The water evaporated from the discs and the discs were re-weighed on a microbalance to obtain the amount of the bioactive agent on the disc. The polymer coating solution containing poly(ethylene-co-vinylacetate) and poly(n-butyl methacrylate) was applied to the entire surface of the disc, covering the dextran. The polymer coating solution was coated under a range of humidity conditions. The tetrahydrofuran evaporated from the discs and the coatings were dried under vacuum. The discs were weighed a third time to obtain the amount of the polymer coating per disc.

### Bioactive Agent Release Assay

A suitable Bioactive Agent Release Assay, as describe herein, can be used to determine the extent and rate of bioactive agent release under physiological conditions. In general it is desirable that less than 50 % of the total quantity of the bioactive agent be released in the first 24 hours. It is frequently desirable for quantities of bioactive agent to be released for a duration of at least 30 days.

Except as otherwise provided herein, each coated disc was placed in an amber vial with 4 mls of PBS. The vials were placed in a water bath and stirred at 37 °C. At time intervals, the disc was removed from the vial, placed into a new vial containing fresh PBS and the new vial was placed into the water bath to continue the experiment. The concentration of bioactive agent in each PBS sample was determined using UV spectroscopy.

After all measurable bioactive agent was released from the coated disc; the disc was washed with water, dried, and re-weighed to determine the weight loss of the disc.

### Conclusion

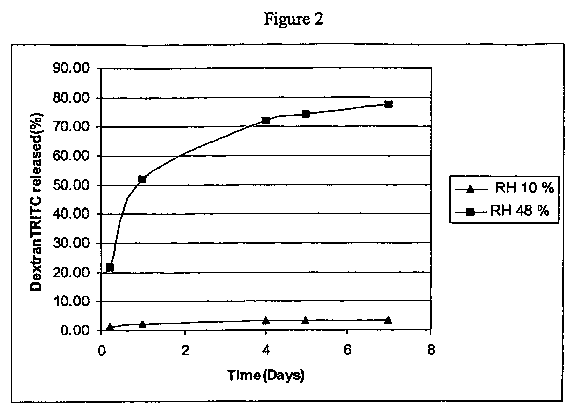

The results are provided in Figure 2 below, where it can be seen that the relative humidity at which the polymeric topcoat composition was coated can be used to control the release rate of the bioactive agent coated in an underlying layer. The bioactive agent was released at a faster rate from the composite coating where the topcoat was coated at 48 % relative humidity than from the polymer topcoat coating that was coated at 10 % relative humidity.

### Reference EXAMPLE 3

### Solution Preparation β-estradiol

A polymer coating solution containing poly(ethylene-co-vinylacetate) with a vinyl acetate concentration of 33 % (w/w) and poly(n-butyl methacrylate) was prepared in tetrahydrofuran at a polymer ratio of 14/86 % (w/w), respectively. β-estradiol was added to the polymer coating solution after dissolution of the polymer in order to provide a coating composition of Applicant's above-captioned copending application in the form a one part system. The bioactive agent/polymer ratio of the β-estradiol containing polymer solution was 30/70 % (w/w) at a concentration of 10 mg/ml.

### Sample preparation

Eighteen-millimeter long, electropolished stainless steel stents with a 2 mm outer diameter were fabricated (Laserage Technology Corporation, Waukegan IL). A surface treatment such as Parylene could be applied to the stent or the surface could be left untreated. The stents were weighed on a microbalance. The β-estradiol containing polymer solution was coated (e.g., sprayed) onto stainless steel stents in an environment maintained at 0, 20, 30 or 40 % relative humidity at 22 °C. The stents were re-weighed after drying on a microbalance to obtain the amount of the β-estradiol per stent.

### Bioactive Agent Release Assay

A suitable Bioactive Agent Release Assay, as described herein, can be used to determine the extent and rate of bioactive agent release under physiological conditions. In general it is desirable that less that 50 % of the total quantity of the bioactive agent be released in the first 24 hours. It is frequently desirable for quantities of bioactive agent to be released for a duration of at least 30 days.

Except as otherwise provided herein, each coated stent was placed in an amber vial with 1.6 mls of PBS. The vials were placed in a water bath and stirred at 37 °C. At time intervals, the stent was removed from the vial, placed into a new vial containing fresh PBS and the new vial was placed into the water bath to continue the experiment. The concentration of β-estradiol in each PBS sample was determined using UV spectroscopy.

### Conclusion

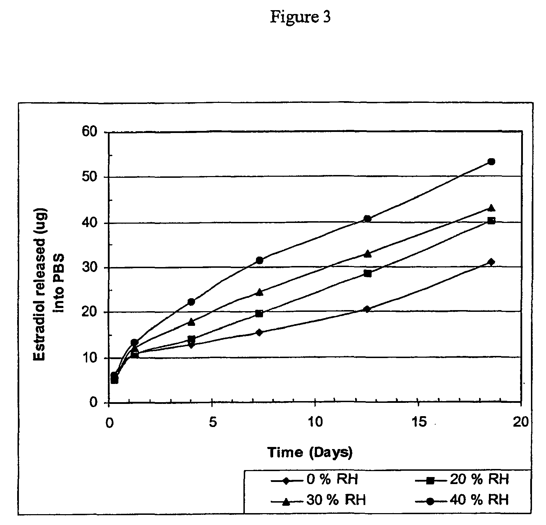

The results are provided in Figure 3 below, where it can be seen that the coating of the stents under different humidity level conditions can be used to control the β-estradiol rate of release from coatings containing poly(ethylene-co-vinylacetate) and poly(n-butyl methacrylate).

## Claims

1. A coating composition comprising a bioactive agent in combination with a plurality of polymers, including a first polymer component comprising at least one aromatic poly(meth)acrylate polymer and a second polymer component comprising poly(ethylene-co-vinyl acetate).

2. A composition according to claim 1 wherein the first polymer composition comprises a polymer selected from the group consisting of polyaryl(meth)acrylates, polyaralkyl(meth)acrylates, and polyaryloxyalkyl(meth)acrylates.

3. A composition according to claim 1 wherein the first polymer component is selected from the group consisting of: polyaryl(meth)acrylates, polyarallcyl(meth)acrylates, and polyaryloxyallcyl(meth)acrylates with aryl groups having from 6 to 16 carbon atoms, having a weight average molecular weight of 50 to 900 kilodaltons.

4. A composition according to claim 2 wherein the polyaryl(meth)acrylates are selected from the group consisting of poly-9-anthracenylmethacrylate, polychlorophenylacrylate, polymethacryloxy-2-hydroxybenzophenone, polymethacryloxybenzotriazole, polynaphthylacrylate, polynaphthylmethacrylate, poly-4-nitrophenylacrylate, polypentachloro(bromo, fluoro)acrylate and methacrylate, polyphenylacrylate and methacrylate, the polyaralkyl(meth)acrylates are selected from the group consisting of polybenzylacrylate and methacrylate, poly-2-phenethylacrylate and methacrylate, poly-1-pyrenylmethylmethacrylate, and the polyaryloxyalkyl(meth)acrylates are selected from the group consisting of polyphenoxyethylacrylate and methacrylate, polyethyleneglycolphenylether acrylates and methacrylates.

5. A composition according to claim 2 wherein the second polymer component is selected from the group consisting of poly(ethylene-co-vinyl acetate) polymers having vinyl acetate concentrations of between 8 % and 90 % by weight.

6. A composition according to claim 5 wherein the vinyl acetate concentrations are between 20 % and 40 % by weight.

7. A composition according to claim 1 wherein the composition is provided in a form selected from the group of solution, emulsion, mixture, dispersion or blend.

8. A composition according to claim 7 wherein the total combined concentrations of both polymers in the composition is between 0.05 % and 70 % by weight.

9. A composition according to claim 2 wherein the first polymeric component has a weight average molecular weight of from 100 kilodaltons to 500 kilodaltons and the poly(ethylene-co-vinyl acetate) has a vinyl acetate content of from 20 % to 40 % by weight.

10. A composition according to claim 9 wherein the bioactive agent is dissolved or suspended in the coating composition at a concentration of 0.01 % to 90 % by weight and is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives, anticancer chemotherapeutic agents, anti-inflammatory steroid or non-steroidal anti-inflammatory agents, immunosuppressive agents, growth hormone antagonists, growth factors, dopamine agonists, radiotherapeutic agents, peptides, proteins, enzymes, extracellular matrix components, inhibitors, free radical scavengers, chelators, antioxidants, anti polymerases, antiviral agents, photodynamic therapy agents, and gene therapy agents.

11. A method of coating a device with a bioactive agent, the method comprising the steps of providing a composition according to claim 1 and applying the composition to the device.

12. A method according to claim 11 wherein the coating is provided upon a surface of an implanted medical device under conditions in which humidity is controlled either by controlling the humidity at which the device is coated with the composition and/or by controlling the water content of the coating or coated composition itself.

13. A method according to claim 11 wherein the composition is provided upon a surface of an implanted medical device and comprises a plurality of coating compositions, each independently coated under conditions of controlled humidity.

14. A method according to claim 11 wherein the device is one that undergoes flexion and/or expansion in the course of implantation or use *in vivo*.

15. A method according to claim 11 wherein the first polymer component is selected from the group consisting of: polyaryl(meth)acrylates, polyaralkyl(meth)acrylates, and polyaryloxyalkyl(meth)acrylates with aryl groups having from 6 to 16 carbon atoms, having a weight average molecular weight of 50 to 900 kilodaltons.

16. A method according to claim 15 wherein the polyaryl(meth)acrylates are selected from the group consisting of poly-9-anthracenylmethacrylate, polychlorophenylacrylate, polymethacryloxy-2-hydroxybenzophenone, polymethacryloxybenzotriazole, polynaphthylacrylate, polynaphthylmethacrylate, poly-4-nitrophenylacrylate, polypentachloro(bromo, fluoro)acrylate and methacrylate, polyphenylacrylate and methacrylate, the polyaralkyl(meth)acrylates are selected from the group consisting of polybenzylacrylate and methacrylate, poly-2-phenethylacrylate and methacrylate, poly-1-pyrenylmethylmethacrylate, and the polyaryloxyalkyl(meth)acrylates are selected from the group consisting of polyphenoxyethylacrylate and methacrylate, polyethyleneglycolphenylether acrylates and methacrylates.

17. A method according to claim 12 wherein the coating composition is coated onto the device under relative humidity controlled at a level of between 0 % and 95 % relative humidity.

18. A method according to claim 11 wherein the second polymer component is selected from the group consisting of poly(ethylene-co-vinyl acetate) polymers having vinyl acetate concentrations of between 8 % and 90 % by weight.

19. A method according to claim 18 wherein the vinyl acetate concentrations are between 20 % and 40 % by weight.

20. A method according to claim 11 wherein the composition is provided in a form selected from the group of solution, emulsion, mixture, dispersion or blend.

21. A method according to claim 20 wherein the total combined concentrations of both polymers in the composition is between 0.05 % and 70 % by weight.

22. A method according to claim 20 wherein the first polymeric component has a weight average molecular weight of from 100 kilodaltons to 500 kilodaltons and the poly(ethylene-*co*-vinyl acetate) has a vinyl acetate content of from 20 % to 40 % by weight.

23. A method according to claim 22 wherein the first polymeric component has a weight average molecular weight of from 200 kilodaltons to 400 kilodaltons and the poly(ethylene-co-vinyl acetate) has a vinyl acetate content of from 30 % to 34 % by weight.

24. A method according to claim 11 wherein the bioactive agent is dissolved or suspended in the coating composition at a concentration of 0.01 % to 90 % by weight.

25. A method according to claim 24 wherein the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, ailtiplatelet agents, antimitotics, microtubule inhibitors, anti secretory agents, actin inhibitors, remodeling inhibitors, antisense nucleotides, anti metabolites, antiproliferatives, anticancer chemotherapeutic agents, anti-inflammatory steroid or non-steroidal anti-inflammatory agents, immunosuppressive agents, growth hormone antagonists, growth factors, dopamine agonists, radiotherapeutic agents, peptides, proteins, enzymes, extracellular matrix components, inhibitors, free radical scavengers, chelators, antioxidants, anti polymerases, antiviral agents, photodynamic therapy agents, and gene therapy agents.

26. A method according to claim 12 wherein the bioactive agent is dissolved or suspended in the coating composition at a concentration of 0.01 % to 90 % by weight.

27. A method according to claim 26 wherein the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives, anticancer chemotherapeutic agents, anti-inflammatory steroid or non-steroidal anti-inflammatory agents, immunosuppressive agents, growth hormone antagonists, growth factors, dopamine agonists, radiotherapeutic agents, peptides, proteins, enzymes, extracellular matrix components, inhibitors, free radical scavengers, chelators, antioxidants, anti polymerases, antiviral agents, photodynamic therapy agents, and gene therapy agents.

28. A method according to claim 18 wherein the bioactive agent is dissolved or suspended in the coating composition at a concentration of 0.01 % to 90 % by weight.

29. A method according to claim 28 wherein the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives, anticancer chemotherapeutic agents, anti-inflammatory steroid or non-steroidal anti-inflammatory agents, immunosuppressive agents, growth hormone antagonists, growth factors, dopamine agonists, radiotherapeutic agents, peptides, proteins, enzymes, extracellular matrix components, inhibitors, free radical scavengers, chelators, antioxidants, anti polymerases, antiviral agents, photodynamic therapy agents, and gene therapy agents.

30. A combination comprising a device coated with a composition according to the method of claim 11, the combination being adapted to provide controlled release of the bioactive agent when positioned in an aqueous environment.

31. A combination according to claim 30 wherein the device is an implantable medical device that that undergoes flexion and/or expansion in the course of implantation or use *in vivo,* and the surface is coated with a plurality of coating compositions, each independently coated under conditions of controlled humidity.

32. A combination according to claim 30 wherein the first polymer component is selected from the group consisting of polyaryl(meth)acrylates, polyaralkyl(meth)acrylates, and polyaryloxyallcyl(meth)acrylates with aryl groups having from 6 to 16 carbon atoms, having a weight average molecular weight of 50 to 900 kilodaltons,
and the second polymer component is selected from the group consisting of poly(ethylene-co-vinyl acetate) polymers having vinyl acetate concentrations of between 8 % and 90 % by weight.

33. A combination according to claim 32 wherein the total combined concentrations of both polymers in the composition is between 0.05 % and 70 % by weight, and the bioactive agent is dissolved or suspended in the coating composition at a concentration of 0.01 % to 90 % by weight.

34. A combination according to claim 33 wherein the device is selected from the group consisting of catheters and stents.

35. A combination according to claim 34 wherein the catheter is selected from the group consisting of urinary catheters and intravenous catheters.

36. A combination according to claim 30 wherein the weight of the coating attributable to the bioactive agent is in the range of one microgram to 10 mg of bioactive agent per cm² of the gross surface area of the device.

37. A combination according to claim 36 wherein the weight of the coating attributable to the bioactive agent is between 0.01 mg and 0.5 mg of bioactive agent per cm² of the gross surface area of the device, and the coating thickness of the composition is in the range of 0.1 micrometers to 100 micrometers.

38. The combination of claims 30 to 37 for use in therapy.

39. A composition according to one of claims 1 or 3 wherein the composition further comprises a solvent in which the polymers form a true solution.

40. A method according to one of claims 11 or 22 wherein the device comprises a biomaterial selected from the group consisting of acrylics, vinyls, nylons, polyurethanes, polycarbonates, polyamides, polysulfones, poly(ethylene terephthalate), polylactic acid, polyglycolic acid, polydimethylsiloxanes, and polyetheretherketones, natural organic materials, metals, ceramics, glass, silica, and sapphire.

41. A method according to claim 40 wherein the acrylics are selected from methyl acrylate, methyl methacrylate, hydroxyethyl methacrylate, hydroxyethyl acrylate, acrylic acid, methacrylic acid, glyceryl acrylate, glyceryl methacrylate, methacrylamide, and acrylamide, the vinyls are selected from ethylene, propylene, styrene, vinyl.chloride, vinyl acetate, vinyl pyrrolidone, and vinylidene difluoride, the nylons are selected from polycaprolactam, polylauryl lactam, polyhexamethylene adipamide, and polyhexamethylene dodecanediamide, the organic materials are selected from human tissue, wood, cellulose, compressed carbon, and rubber, the metals are selected from titanium, stainless steel, cobalt chromium, gold, silver, copper, and platinum and their alloys, and the ceramics are selected from silicon nitride, silicon carbide, zirconia, and alumina, including combinations of such biomaterials.

42. A method according to one of claims 11 or 22 wherein the device is selected from the group consisting of vascular devices, orthopedic devices, dental devices, drug delivery devices, ophthalmic devices, glaucoma drain shunts, urological devices, synthetic prostheses, dialysis tubing and membranes, blood oxygenator tubing and membranes, blood bags, sutures, membranes, cell culture devices, chromatographic support materials, and biosensors.

43. A method according to claim 42 wherein the vascular devices are selected from grafts, stents, catheters, valves, artificial hearts, and heart assist devices, the orthopedic devices are selected from joint implants, fracture repair devices, and artificial tendons, the dental devices are selected from dental implants and fracture repair devices, and the urological devices are selected from penile, sphincter, urethral, bladder, and renal devices.

44. A composition according to any one of Claims 1-9 and 39, wherein the bioactive agent is an antiproliferative.

45. A composition according to any one of Claims 1-9 and 39, wherein the bioactive agent is β-estradiol.

46. A method according to any one of Claims 11-24, 26, 28, wherein the bioactive agent is an antiproliferative.

47. A method according to any one of Claims 11-24, 26, 28, wherein the bioactive agent is β-estradiol.

48. A combination according to any one of Claims 30-38, wherein the bioactive agent is an antiproliferative.

49. A combination according to any one of Claims 30-38, wherein the bioactive agent is β-estradiol.

## Patentansprüche

1. Beschichtungszusammensetzung, die einen bioaktiven Wirkstoff umfaßt, in Kombination mit mehreren Polymeren, einschließlich einer ersten Polymerkomponente, die wenigstens ein aromatisches Poly(meth)acrylat-Polymer umfaßt, und einer zweiten Polymerkomponente, die ein Poly(ethylen-co-vinylacetat) umfaßt.

2. Zusammensetzung nach Anspruch 1, **dadurch gekennzeichnet, daß** die erste Polymerzusammensetzung ein Polymer umfaßt, das ausgewählt ist aus der Gruppe, die aus Polyaryl(meth)acrylaten, Polyaralkyl(meth)acrylaten und Polyaryloxyalkyl(meth)-acrylaten besteht.

3. Zusammensetzung nach Anspruch 1, **dadurch gekennzeichnet, daß** die erste Polymerkomponente ausgewählt ist aus der Gruppe, die aus: Polyaryl(meth)acrylaten, Polyaralkyl(meth)acrylaten und Polyaryloxyalkyl(meth)acrylaten besteht, mit Arylgruppen mit 6 bis 16 Kohlenstoffatomen, mit einem gewichtsgemittelten Molekulargewicht von 50 bis 900 Kilodaltons.

4. Zusammensetzung nach Anspruch 2, **dadurch gekennzeichnet, daß** die Polyaryl(meth)acrylate ausgewählt sind aus der Gruppe, die aus Poly-9-anthracenylmethacrylat, Polychlorphenylacrylat, Polymethacryloxy-2-hydroxybenzophenon, Polymethacryloxybenzotriazol, Polynaphthylacrylat, Polynaphthylmethacrylat, Poly-4-nitrophenylacrylat, Polypentachlor(brom,fluor)acrylat und -methacrylat, Polyphenylacrylat und -methacrylat besteht, die Polyaralkyl(meth)acrylate ausgewählt sind aus der Gruppe, die aus Polybenzylacrylat und -methacrylat, Poly-2-phenethylacrylat und -methacrylat, Poly-1-pyrenylmethylmethacrylat besteht, und die Polyaryloxyalkyl(meth)acrylate ausgewählt sind aus der Gruppe, die aus Polyphenoxyethylacrylat und -methacrylat, Polyethylenglykolphenyletheracrylaten und -methacrylaten besteht.

5. Zusammensetzung nach Anspruch 2, **dadurch gekennzeichnet, daß** die zweite Polymerkomponente ausgewählt ist aus der Gruppe, die aus Poly(ethylen-co-vinylacetat)-Polymeren mit Vinylacetat-Konzentrationen von zwischen 8 und 90 Gew.-% besteht.

6. Zusammensetzung nach Anspruch 5, **dadurch gekennzeichnet, daß** die Vinylacetat-Konzentrationen zwischen 20 und 40 Gew.-% liegen.

7. Zusammensetzung nach Anspruch 1, **dadurch gekennzeichnet, daß** die Zusammensetzung in einer Form bereitgestellt ist, die ausgewählt ist aus der Gruppe Lösung, Emulsion, Mischung, Dispersion oder Gemisch.

8. Zusammensetzung nach Anspruch 7, **dadurch gekennzeichnet, daß** die kombinierte Gesamtkonzentration beider Polymere in der Zusammensetzung zwischen 0,05 und 70 Gew.-% liegt.

9. Zusammensetzung nach Anspruch 2, **dadurch gekennzeichnet, daß** die erste polymere Komponente ein gewichtsgemitteltes Molekulargewicht von 100 Kilodaltons bis 500 Kilodaltons besitzt und das Poly(ethylen-co-vinylacetat) einen Vinylacetat-Gehalt von 20 bis 40 Gew.-% besitzt.

10. Zusammensetzung nach Anspruch 9, **dadurch gekennzeichnet, daß** der bioaktive Wirkstoff in der Beschichtungszusammensetzung in einer Konzentration von 0,01 bis 90 Gew.-% gelöst oder suspendiert ist und ausgewählt ist aus der Gruppe, die aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretorischen Mitteln, Actin-Inhibitoren, Remodeling-Inhibitoren, Antisense-Nukleotiden, Antimetaboliten, Antiproliferativa, chemotherapeutischen Antikrebsmitteln, entzündungshemmenden steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Inhibitoren, Radikalfängern, Chelatbildnern; Antioxidationsmitteln, Antipolymerasen, antiviralen Mitteln, Mitteln für photodynamische Therapie und Mitteln für Gentherapie besteht.

11. Verfahren zum Beschichten einer Vorrichtung mit einem bioaktiven Wirkstoff, wobei das Verfahren die Schritte der Bereitstellung der Zusammensetzung nach Anspruch 1 und das Aufbringen der Zusammensetzung auf die Vorrichtung umfaßt.

12. Verfahren nach Anspruch 11, **dadurch gekennzeichnet, daß** die Beschichtung auf einer Oberfläche einer implantierten medizinischen Vorrichtung unter Bedingungen bereitgestellt wird, bei denen die Feuchtigkeit entweder durch Kontrollieren der Feuchtigkeit, bei der die Vorrichtung mit der Zusammensetzung beschichtet wird, und/oder durch Kontrollieren des Wassergehaltes der Beschichtung oder beschichteten Zusammensetzung selbst kontrolliert wird.

13. Verfahren nach Anspruch 11, **dadurch gekennzeichnet, daß** die Zusammensetzung auf einer Oberfläche einer implantierten medizinischen Vorrichtung bereitgestellt wird und mehrere Beschichtungszusammensetzungen umfaßt, die jede unabhängig unter Bedingungen kontrollierter Feuchtigkeit aufgebracht werden.

14. Verfahren nach Anspruch 11, **dadurch gekennzeichnet, daß** die Vorrichtung eine ist, die im Verlaufe der Implantation oder der Verwendung in vivo eine Biegung und/oder Expansion durchläuft.

15. Verfahren nach Anspruch 11, **dadurch gekennzeichnet, daß** die erste Polymerkomponente ausgewählt ist aus der Gruppe, die aus: Polyaryl(meth)acrylaten, Polyaralkyl(meth)acrylaten und Polyaryloxyalkyl(meth)acrylaten besteht, mit Arylgruppen mit von 6 bis 16 Kohlenstoffatomen, mit einem gewichtsgemittelten Molekulargewicht von 50 bis 900 Kilodaltons.

16. Verfahren nach Anspruch 15, **dadurch gekennzeichnet, daß** die Polyarl(meth)acrylate ausgewählt sind aus der Gruppe, die aus Poly-9-anthracenylmethacrylat, Polychlorphenylacrylat, Polymethacryloxy-2-hydroxybenzophenon, Polymethacryloxybenzotriazol, Polynaphthylacrylat, Polynaphthylmethacrylat, Poly-4-nitrophenylacrylat, Polypentachlor(brom,fluor)acrylat und -methacrylat, Polyphenylacrylat und -methacrylat besteht, die Polyaralkyl(meth)acrylate ausgewählt sind aus der Gruppe, die aus Polybenzylacrylat und -methacrylat, Poly-2-phenethylacrylat und -methacrylat, Poly-1-pyrenylmethylmethacrylat besteht, und die Polyaryloxyalkyl(meth)acrylate ausgewählt sind aus der Gruppe, die aus Polyphenoxyethylacrylat und -methacrylat, Polyethylenglykolphenyletheracrylaten und -methacrylaten besteht.

17. Verfahren nach Anspruch 12, **dadurch gekennzeichnet, daß** die Beschichtungszusammensetzung auf die Vorrichtung unter einer relativen Feuchtigkeit aufgebracht wird, die auf ein Niveau von zwischen 0 und 95% relativer Feuchtigkeit eingestellt wird.

18. Verfahren nach Anspruch 11, **dadurch gekennzeichnet, daß** die zweite Polymerkomponente ausgewählt ist aus der Gruppe, die aus Poly(ethylen-co-vinylacetat)-Polymeren mit Vinylacetat-Konzentrationen von zwischen 8 und 90 Gew.-% besteht.

19. Verfahren nach Anspruch 18, **dadurch gekennzeichnet, daß** die Vinylacetat-Konzentrationen zwischen 20 und 40 Gew.-% liegen.

20. Verfahren nach Anspruch 11, **dadurch gekennzeichnet, daß** die Zusammensetzung in einer Form bereitgestellt wird, die ausgewählt ist aus der Gruppe Lösung, Emulsion, Mischung, Dispersion oder Gemisch.

21. Verfahren nach Anspruch 20, **dadurch gekennzeichnet, daß** die kombinierte Gesamtkonzentration beider Polymere in der Zusammensetzung zwischen 0,05 und 70 Gew.-% liegt.

22. Verfahren nach Anspruch 20, **dadurch gekennzeichnet, daß** die erste polymere Komponente ein gewichtsgemitteltes Molekulargewicht von 100 Kilodaltons bis 500 Kilodaltons besitzt und das Poly(ethylen-co-vinylacetat) einen Vinylacetat-Gehalt von 20 bis 40 Gew.-% besitzt.

23. Verfahren nach Anspruch 22, **dadurch gekennzeichnet, daß** die erste polymere Komponente ein gewichtsgemitteltes Molekulargewicht von 200 Kilodaltons bis 400 Kilodaltons besitzt und das Poly(ethylen-co-vinylacetat) einen Vinylacetat-Gehalt von 30 bis 34 Gew.-% besitzt.

24. Verfahren nach Anspruch 11, **dadurch gekennzeichnet, daß** der bioaktive Wirkstoff in der Beschichtungszusammensetzung in einer Konzentration von 0,01 bis 90 Gew.-% gelöst oder suspendiert wird.

25. Verfahren nach Anspruch 24, **dadurch gekennzeichnet, daß** der bioaktive Wirkstoff ausgewählt ist aus der Gruppe, die aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretorischen Mitteln, Actin-Inhibitoren, Remodeling-Inhibitoren, Antisense-Nukleotiden, Antimetaboliten, Antiproliferativa, chemotherapeutischen Antikrebsmitteln, entzündungshemmenden steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Inhibitoren, Radikalfängern, Chelatbildnern, Antioxidationsmitteln, Antipolymerasen, antiviralen Mitteln, Mitteln für photodynamische Therapie und Mitteln für Gentherapie besteht.

26. Verfahren nach Anspruch 12, **dadurch gekennzeichnet, daß** der bioaktive Wirkstoff in der Beschichtungszusammensetzung in einer Konzentration von 0,01 bis 90 Gew.-% gelöst oder suspendiert wird.

27. Verfahren nach Anspruch 26, **dadurch gekennzeichnet, daß** der bioaktive Wirkstoff ausgewählt ist aus der Gruppe, die aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretorischen Mitteln, Actin-Inhibitoren, Remodeling-Inhibitoren, Antisense-Nukleotiden, Antimetaboliten, Antiproliferativa, chemotherapeutischen Antikrebsmitteln, entzündungshemmenden steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Inhibitoren, Radikalfängern, Chelatbildnern, Antioxidationsmitteln, Antipolymerasen, antiviralen Mitteln, Mitteln für photodynamische Therapie und Mitteln für Gentherapie besteht.

28. Verfahren nach Anspruch 18, **dadurch gekennzeichnet, daß** der bioaktive Wirkstoff in der Beschichtungszusammensetzung in einer Konzentration von 0,01 bis 90 Gew.-% gelöst oder suspendiert wird.

29. Verfahren nach Anspruch 28, **dadurch gekennzeichnet, daß** der bioaktive Wirkstoff ausgewählt ist aus der Gruppe, die aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretorischen Mitteln, Actin-Inhibitoren, Remodeling-Inhibitoren, Antisense-Nukleotiden, Antimetaboliten, Antiproliferativa, chemotherapeutischen Antikrebsmitteln, entzündungshemmenden steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Inhibitoren, Radikalfängern, Chelatbildnern, Antioxidationsmitteln, Antipolymerasen, antiviralen Mitteln, Mitteln für photodynamische Therapie und Mitteln für Gentherapie besteht.

30. Kombination, die eine Vorrichtung umfaßt, die mit einer Zusammensetzung nach dem Verfahren von Anspruch 11 beschichtet ist, wobei die Kombination angepaßt ist, um kontrollierte Freisetzung des bioaktiven Wirkstoffes bereitzustellen, wenn sie in einer wäßrigen Umgebung positioniert ist.

31. Kombination nach Anspruch 30, **dadurch gekennzeichnet, daß** die Vorrichtung eine implantierbare medizinische Vorrichtung ist, die im Verlaufe der Implantation oder der Verwendung in vivo eine Biegung und/oder Expansion durchläuft, und die Oberfläche mit mehreren Beschichtungszusammensetzungen beschichtet ist, die jede unabhängig unter Bedingungen kontrollierter Feuchtigkeit aufgebracht sind.

32. Kombination nach Anspruch 30, **dadurch gekennzeichnet, daß** die erste Polymerkomponente ausgewählt ist aus der Gruppe, die aus Polyaryl(meth)acrylaten, Polyaralkyl(meth)acrylaten und Polyaryloxyalkyl(meth)acrylaten besteht, mit Arylgruppen mit von 6 bis 16 Kohlenstoffatomen, mit einem gewichtsgemittelten Molekulargewicht von 50 bis 900 Kilodaltons,
und die zweite Polymerkomponente ausgewählt ist aus der Gruppe, die aus Poly(ethylen-co-vinylacetat)-Polymeren mit Vinylacetat-Konzentrationen von zwischen 8 und 90 Gew.-% besteht.

33. Kombination nach Anspruch 32, **dadurch gekennzeichnet, daß** die kombinierte Gesamtkonzentration beider Polymere in der Zusammensetzung zwischen 0,05 und 70 Gew.-% liegt und der bioaktive Wirkstoff in der Beschichtungszusammensetzung in einer Konzentration von 0,01 bis 90 Gew.-% gelöst oder suspendiert ist.

34. Kombination nach Anspruch 33, **dadurch gekennzeichnet, daß** die Vorrichtung ausgewählt ist aus der Gruppe, die aus Kathetern und Stents besteht.

35. Kombination nach Anspruch 34, **dadurch gekennzeichnet, daß** der Katheter ausgewählt ist aus der Gruppe, die aus 1-iarnblasenkathetern und intravenösen Kathetern besteht.

36. Kombination nach Anspruch 30, **dadurch gekennzeichnet, daß** das Gewicht der Beschichtung, das dem bioaktiven Wirkstoff zuschreibbar ist, im Bereich von einem Mikrogramm bis 10 mg bioaktiver Wirkstoff pro cm² der Bruttooberfläche der Vorrichtung liegt.

37. Kombination nach Anspruch 36, **dadurch gekennzeichnet, daß** das Gewicht der Beschichtung, das dem bioaktiven Wirkstoff zuschreibbar ist, zwischen 0,01 mg und 0,5 mg bioaktiver Wirkstoff pro cm² der Bruttooberfläche der Vorrichtung liegt und die Beschichtungsdicke der Zusammensetzung im Bereich von 0,1 Mikrometern bis 100 Mikrometern liegt.

38. Kombination nach den Ansprüchen 30 bis 37 zur Verwendung in der Therapie.

39. Zusammensetzung nach einem der Ansprüche 1 oder 3, **dadurch gekennzeichnet, daß** die Zusammensetzung weiter ein Lösemittel umfaßt, in dem die Polymere eine echte Lösung bilden.

40. Verfahren nach einem der Ansprüche 11 oder 22, **dadurch gekennzeichnet, daß** die Vorrichtung ein Biomaterial umfaßt, das ausgewählt ist aus der Gruppe, die aus Acrylverbindungen, Vinyls, Nylons, Polyurethanen, Polycarbonaten, Polyamiden, Polysulfonen, Poly(ethylenterephthalat), Polymilchsäure, Polyglykolsäure, Polydimethylsiloxanen und Polyetheretherketonen, natürlichen organischen Materialien, Metallen, Keramikwerkstoffen, Glas, Siliciumdioxid und Saphir besteht.

41. Verfahren nach Anspruch 40, **dadurch gekennzeichnet, daß** die Acrylverbindungen ausgewählt sind aus Methylacrylat, Methylmethacrylat, Hydroxyethylmethacrylat, Hydroxyethylacrylat, Acrylsäure, Methacrylsäure, Glycerylacrylat, Glycerylmethacrylat, Methacrylamid und Acrylamid, die Vinyls ausgewählt sind aus Ethylen, Propylen, Styrol, Vinylchlorid, Vinylacetat, Vinylpyrrolidon und Vinylidendifluorid, die Nylons ausgewählt sind aus Polycaprolactam, Polylauryllactam, Polyhexamethylenadipamid und Polyhexamethylendodecandiamid, die organischen Materialien ausgewählt sind aus menschlichem Gewebe, Holz, Cellulose, verdichtetem Kohlenstoff und Gummi, die Metalle ausgewählt sind aus Titan, rostfreiem Stahl, Cobaltchrom, Gold, Silber, Kupfer und Platin und deren Legierungen und die Keramikwerkstoffe ausgewählt sind aus Siliciumnitrid, Silciumcarbid, Zirconiumoxid und Aluminumoxid, einschließlich Kombinationen solcher Biomaterialien.

42. Verfahren nach einem der Ansprüche 11 oder 22, **dadurch gekennzeichnet, daß** die Vorrichtung ausgewählt ist aus der Gruppe, die aus vaskulären Vorrichtungen, orthopädischen Vorrichtungen, dentalen Vorrichtungen, Arzneistoffabgabevorrichtungen, ophtalmischen Vorrichtungen, Glaukomdrainageshunts, urologischen Vorrichtungen, synthetischen Prothesen, Dialyseschläuchen und -membranen, Blutoxygenatorschläuchen und -membranen, Blutbeuteln, Nahtmaterialien, Membranen, Zellkulturvorrichtungen, Chromatographieträgermaterialien und Biosensoren besteht.

43. Verfahren nach Anspruch 42, **dadurch gekennzeichnet, daß** die vaskulären Vorrichtungen ausgewählt sind aus Transplantaten, Stents, Kathetern, Herzklappen, künstlichen Herzen und Herzunterstützungsvorrichtungen, die orthopädischen Vorrichtungen ausgewählt sind aus Gelenkimplantaten, Frakturreparaturvorrichtungen und künstlichen Sehnen, die dentalen Vorrichtungen ausgewählt sind aus dentalen Implantaten und Frakturreparaturvorrichtungen und die urologischen Vorrichtungen ausgewählt sind aus Penis-, Schließmuskel-, Harnweg-, Blasen- und Nierenvorrichtungen.

44. Zusammensetzung nach einem der Ansprüche 1-9 und 39, **dadurch gekennzeichnet, daß** der bioaktive Wirkstoff ein Antiproliferativum ist.

45. Zusammensetzung nach einem der Ansprüche 1-9 und 39, **dadurch gekennzeichnet, daß** der bioaktive Wirkstoff β-Estradiol ist.

46. Verfahren nach einem der Ansprüche 11-24, 26, 28, **dadurch gekennzeichnet, daß** der bioaktive Wirkstoff ein Antiproliferativum ist.

47. Verfahren nach einem der Ansprüche 11-24, 26, 28, **dadurch gekennzeichnet, daß** der bioaktive Wirkstoff β-Estradiol ist.

48. Kombination nach einem der Ansprüche 30-38, **dadurch gekennzeichnet, daß** der bioaktive Wirkstoff ein Antiproliferativum ist.

49. Kombination nach einem der Ansprüche 30-38, **dadurch gekennzeichnet, daß** der bioaktive Wirkstoff β-Estradiol ist.

## Revendications

1. Composition de revêtement comprenant un agent bioactif en combinaison avec plusieurs polymères, incluant un premier composant polymère comprenant au moins un polymère poly(mëth)acrylate aromatique et un second composant polymère comprenant du poly(éthylène-co-acétate de vinyle).

2. Composition selon la revendication 1, dans laquelle la première composition polymère comprend un polymère choisi dans le groupe constitué par les poly(méth)acrylates d'aryle, des poly(méth)acrylates d'aralkyle, et les poly(méth)acrylates d'aryloxyalkyle.

3. Composition selon la revendication 1, dans laquelle le premier composant polymère est choisi dans le groupe constitué par des poly(méth)acrylates d'aryle, des poly(méth)acrylates d'aralkyle et des poly(méth)acrylates d'aryloxyalkyle avec des groupes aryle, ayant de 6 à 16 atomes de carbone, ayant un poids moléculaire moyen en poids de 50 à 900 kilodaltons.

4. Composition selon la revendication 2, dans laquelle les poly(méth)acrylates d'aryle sont choisis dans le groupe constitué par le poly(méth)acrylate de 9-anthracényle, le polychlorophénylacrylate, la polyméthacryloxy-2-hydroxybenzophénone, le polyméthacryloxybenzotriazole, le polynaphtylacrylate, le polynaphtylméthacrylate, le poly-4-nitrophénylacrylate, le polypentachloro(bromo, fluoro)acrylate et méthacrylate, le polyphénylacrylate et méthacrylate, les poly(méth)acrylates d'aralkyle sont choisis dans le groupe constitué par le polybenzylacrylate et méthacrylate, le poly-2-phénéthylacrylate et méthacrylate, le poly-1-pyrénylméthylméthacrylate, et les poly(méth)acrylates d'aryloxyalkyle sont choisis dans le groupe constitué par le polyphénoxyéthylacrylate et méthacrylate, les polyéthylèneglycolphényléther acrylates et méthacrylates.

5. Composition selon la revendication 2, dans laquelle le second composant polymère est choisi dans le groupe constitué par des polymères poly(éthylène-co-acétate de vinyle) ayant des concentrations en acétate de vinyle comprises entre 8% et 90% en poids.

6. Composition selon la revendication 5, dans laquelle les concentrations en acétate de vinyle sont comprises entre 20% et 40% en poids.

7. Composition selon la revendication 1, dans laquelle la composition est mise en oeuvre sous une forme choisie dans le groupe de la solution, de l'émulsion, du mélange, de la dispersion ou du mélange.

8. Composition selon la revendication 7, dans laquelle les concentrations combinées totales des deux polymères dans la composition sont comprises entre 0,05% et 70% en poids.

9. Composition selon la revendication 2, dans laquelle le premier composant polymère a un poids moléculaire moyen en poids de 100 kilodaltons à 500 kilodaltons et le poly(éthylène-co-acétate de vinyle) a une teneur en acétate de vinyle de 20% à 40% en poids.

10. Composition selon la revendication 9, dans laquelle l'agent bioactif est dissous ou mis en suspension dans la composition de revêtement à des concentrations de 0,01% à 90% en poids et est choisi dans le groupe constitué par les inhibiteurs de thrombine, les agents antithrombogènes, les agents thrombolytiques, les agents fibrinolytiques, les inhibiteurs de vasospasme, les agents bloquants les canaux de calcium, les vasodilatateurs, les agents anti-hypertenseurs, les agents antimicrobiens, les antibiotiques, les inhibiteurs des récepteurs des glycoprotéines de surface, les agents anti-plaquettes, les antimitotiques, les inhibiteurs des microtubules, les agents anti-sécrétion, les inhibiteurs de l'actine, les inhibiteurs de remodelage, les nucléotides anti-sens, les anti-métabolites, les agents anti-prolifération, les agents chimiothérapeutiques anticancéreux, les agents anti-inflammatoires stéroïdiens ou les agents anti-inflammatoires anti-stéroïdiens, les agents immunosuppresseurs, les antagonistes de l'hormone de croissance, les facteurs de croissance, les agonistes de la dopamine, les agents radio-thérapeutiques, les peptides, les protéines, les enzymes, les composants de la matrice extracellulaire, les inhibiteurs, les agents de fixation des radicaux libres, les chélatants, les antioxydants, les anti-polymérases, les agents antiviraux, les agents de la thérapie photodynamique, et les agents de la thérapie génique.

11. Procédé de revêtement d'un dispositif avec un agent bioactif, procédé comprenant les étapes de mise en oeuvre d'une composition selon la revendication 1, et d'application de la composition sur le dispositif.

12. Procédé selon la revendication 11, dans lequel le revêtement est mis en oeuvre à la surface d'un dispositif médical implanté dans des conditions dans lesquelles l'humidité est contrôlée soit par contrôle de l'humidité à laquelle le dispositif est revêtu avec la composition et/ou par contrôle de la teneur en eau du revêtement ou de la composition revêtue elle-même.

13. Procédé selon la revendication 11, dans lequel la composition est mise en oeuvre sur une surface d'un dispositif médical implanté et qui comprend plusieurs compositions de revêtement, chacune étant indépendamment revêtue dans des conditions d'humidité contrôlée.

14. Procédé selon la revendication 11, dans lequel le dispositif est l'un qui subit une flexion et/ou une expansion au cours de l'implantation ou de l'utilisation *in vivo.*

15. Procédé selon la revendication 11, dans lequel le premier composant polymère est choisi dans le groupe constitué par des poly(méth)acrylates d'aryle, des poly(méth)acrylates d'aralkyle et des poly(méth)acrylates d'aryloxyalkyle avec des groupes aryle ayant de 6 à 16 atomes de carbone, ayant un poids moléculaire moyen en poids de 50 à 900 kilodaltons.

16. Procédé selon la revendication 15, dans lequel les poly(méth)acrylates d'aryle sont choisis dans le groupe constitué par le poly(méth)acrylate de 9-anthracényle, le polychlorophénylacrylate, la polyméthacryloxy-2-hydroxybenzophénone, le polyméthacryloxybenzotriazole, le polynaphtylacrylate, le polynaphtylméthacrylate, le poly-4-nitrophénylacrylate, le polypentachloro(bromo, fluoro)acrylate et méthacrylate, le polyphénylacrylate et méthacrylate, les poly(méth)acrylates d'aralkyle sont choisis dans le groupe constitué par le polybenzylacrylate et méthacrylate, le poly-2-phénéthylacrylate et méthacrylate, le poly-1-pyrénylméthylméthacrylate, et les poly(méth)acrylates d'aryloxyalkyle sont choisis dans le groupe constitué par le polyphénoxyéthylacrylate et méthacrylate, les polyéthylèneglycolphényléther acrylates et méthacrylates.

17. Procédé selon la revendication 12, dans lequel la composition de revêtement est appliquée en revêtement sur le dispositif sous une humidité relative contrôlée à un niveau entre 0% et 95% d'humidité relative.

18. Procédé selon la revendication 11, dans lequel le second composant polymère est choisi dans le groupe constitué par des polymères poly(éthylène-co-acétate de vinyle) ayant des concentrations en acétate de vinyle comprises entre 8% et 90% en poids.

19. Procédé selon la revendication 18, dans lequel les concentrations en acétate de vinyle sont comprises entre 20% et 40% en poids.

20. Procédé selon la revendication 11, dans lequel la composition est mise en oeuvre sous une forme choisie dans le groupe de la solution, de l'émulsion, du mélange, de la dispersion ou du mélange.

21. Procédé selon la revendication 20, dans lequel les concentrations combinées totales des deux polymères dans la composition sont comprises entre 0,05% et 70% en poids.

22. Procédé selon la revendication 20, dans lequel le premier composant polymère a un poids moléculaire moyen en poids de 100 kilodaltons à 500 kilodaltons et le poly(éthylène-co-acétate de vinyle) a une teneur en acétate de vinyle de 20% à 40% en poids.

23. Procédé selon la revendication 22, dans lequel le premier composant polymère a un poids moléculaire moyen en poids de 200 kilodaltons à 400 kilodaltons et le poly(éthylène-co-acétate de vinyle) a une teneur en acétate de vinyle de 30% à 34% en poids.

24. Procédé selon la revendication 11, dans lequel l'agent bioactif est dissous ou mis en suspension dans la composition de revêtement à une concentration de 0,01% à 90% en poids.

25. Procédé selon la revendication 24, dans lequel l'agent bioactif est choisi dans le groupe constitué par les inhibiteurs de thrombine, les agents antithrombogènes, les agents thrombolytiques, lés agents fibrinolytiques, les inhibiteurs de vasospasme, les agents bloquant les canaux de calcium, les vasodilatateurs, les agents anti-hypertenseurs, les agents antimicrobiens, les antibiotiques, les inhibiteurs des récepteurs des glycoprotéines de surface, les agents anti-plaquettes, les antimitotiques, les inhibiteurs des microtubules, les agents anti-sécrétion, les inhibiteurs de l'actine, les inhibiteurs de remodelage, les nucléotides anti-sens, les anti-métabolites, les agents anti-prolifération, les agents chimiothérapeutiques anticancéreux, les agents anti-inflammatoires stéroïdiens ou les agents anti-inflammatoires anti-stéroïdiens, les agents immunosuppresseurs, les antagonistes de l'hormone de croissance, les facteurs de croissance, les agonistes de la dopamine, les agents radio-thérapeutiques, les peptides, les protéines, les enzymes, les composants de la matrice extracellulaire, les inhibiteurs, les agents de fixation des radicaux libres, les chélatants, les antioxydants, les anti-polymérases, les agents antiviraux, les agents de la thérapie photodynamique, et les agents de la thérapie génique.

26. Procédé selon la revendication 12, dans lequel l'agent bioactif est dissous ou mis en suspension dans la composition de revêtement à une concentration de 0,01% à 90% en poids.

27. Procédé selon la revendication 26, dans lequel l'agent bioactif est choisi dans le groupe constitué par les inhibiteurs de thrombine, les agents antithrombogènes, les agents thrombolytiques, les agents fibrinolytiques, les inhibiteurs de vasospasme, les agents bloquant les canaux de calcium, les vasodilatateurs, les agents anti-hypertenseurs, les agents antimicrobiens, les antibiotiques, les inhibiteurs des récepteurs des glycoprotéines de surface, les agents anti-plaquettes, les antimitotiques, les inhibiteurs des microtubules, les agents anti-sécrétion, les inhibiteurs de l'actine, les inhibiteurs de remodelage, les nucléotides anti-sens, les anti-métabolites, les agents anti-prolifération, les agents chimiothérapeutiques anticancéreux, les agents anti-inflammatoires stéroïdiens ou les agents anti-inflammatoires anti-stéroïdiens, les agents immunosuppresseurs, les antagonistes de l'hormone de croissance, les facteurs de croissance, les agonistes de la dopamine, les agents radio-thérapeutiques, les peptides, les protéines, les enzymes, les composants de la matrice extracellulaire, les inhibiteurs, les agents de fixation des radicaux libres, les chélatants, les antioxydants, les anti-polymérases, les agents antiviraux, les agents de la thérapie photodynamique, et les agents de la thérapie génique.

28. Procédé selon la revendication 18, dans lequel l'agent bioactif est dissous ou mis en suspension dans la composition de revêtement à une concentration de 0,01% à 90% en poids.

29. Procédé selon la revendication 28, dans lequel l'agent bioactif est choisi dans le groupe constitué par les inhibiteurs de thrombine, les agents antithrombogènes, les agents thrombolytiques, les agents fibrinolytiques, les inhibiteurs de vasospasme, les agents bloquant les canaux de calcium, les vasodilatateurs, les agents anti-hypertenseurs, les agents antimicrobiens, les antibiotiques, les inhibiteurs des récepteurs des glycoprotéines de surface, les agents anti-plaquettes, les antimitotiques, les inhibiteurs des microtubules, les agents anti-sécrétion, les inhibiteurs de l'actine, les inhibiteurs de remodelage, les nucléotides anti-sens, les anti-métabolites, les agents anti-prolifération, les agents chimiothérapeutiques anticancéreux, les agents anti-inflammatoires stéroïdiens ou les agents anti-inflammatoires anti-stéroïdiens, les agents immunosuppresseurs, les antagonistes de l'hormone de croissance, les facteurs de croissance, les agonistes de la dopamine, les agents radio-thérapeutiques, les peptides, les protéines, les enzymes, les composants de la matrice extracellulaire, les inhibiteurs, les agents de fixation des radicaux libres, les chélatants, les antioxydants, les anti-polymérases, les agents antiviraux, les agents de la thérapie photodynamique, et les agents de la thérapie génique.

30. Combinaison comprenant un dispositif revêtu avec une composition selon le procédé de la revendication 11, combinaison étant adaptée pour permettre une libération contrôlée de l'agent bioactif quand il est positionné dans un environnement aqueux.

31. Combinaison selon la revendication 30, dans laquelle le dispositif est un dispositif médical implantable qui subit une flexion et/ou une expansion au cours de l'implantation ou de l'utilisation *in vivo,* et la surface est revêtue avec plusieurs compositions de revêtement, chacune étant revêtue indépendamment dans des conditions d'humidité contrôlée.

32. Combinaison selon la revendication 30, dans laquelle le premier composant polymère est choisi dans le groupe constitué par des poly(méth)acrylates d'aryle, des poly(méth)acrylates d'aralkyle et des poly(méth)acrylates d'aryloxyalkyle avec des groupes aryle ayant de 6 à 16 atomes de carbone, et ayant un poids moléculaire moyen en poids de 50 à 900 kilodaltons,
et le second composant polymère est choisi dans le groupe constitué par des polymères poly(éthylène-co-acétate de vinyle) ayant des concentrations en acétate de vinyle comprises entre 8% et 90% en poids.

33. Combinaison selon la revendication 32, dans laquelle les concentrations combinées totales des deux polymères dans la composition sont comprises entre 0,05% et 70% en poids, et l'agent bioactif est dissous ou mis en suspension dans la composition de revêtement à des concentrations de 0,01% à 90% en poids.

34. Combinaison selon la revendication 33, dans laquelle le dispositif est choisi dans le groupe constitué par les cathéters et les stents.

35. Combinaison selon la revendication 34, dans laquelle le cathéter est choisi dans le groupe constitué par des cathéters urinaires et des cathéters intraveineux.

36. Combinaison selon la revendication 30, dans laquelle le poids du revêtement attribuable à l'agent bioactif est dans la plage d'un microgramme à 10 mg d'agent bioactif par cm² de l'aire de surface globale du dispositif.

37. Combinaison selon la revendication 36, dans laquelle le poids du revêtement attribuable à l'agent bioactif est entre 0,01 mg et 0,5 mg d'agent bioactif par cm² de l'aire de surface globale du dispositif, et l'épaisseur du revêtement de la composition est dans la plage de 0,1 micromètre à 100 micromètres.

38. Combinaison des revendications 30 à 37 pour une utilisation en thérapie.

39. Composition selon l'une des revendications 1 ou 3, dans laquelle la composition comprend de plus un solvant dans lequel les polymères forment une solution vraie.

40. Procédé selon l'une des revendications 11 ou 22, dans lequel le dispositif comprend un biomatériau choisi dans le groupe constitué par les acryliques, les vinyles, les nylons, les polyuréthanes, les polycarbonates, les polyamides, les polysulfones, le polyéthylène téréphtalate, le poly(acide lactique), le poly(acide glycolique), les polydiméthylsiloxanes, et les polyétheréthercétones, les matériaux organiques naturels, les métaux, les céramiques, le verre, la silice, et le saphir.

41. Procédé selon la revendication 40, dans lequel les acryliques sont choisis parmi l'acrylate de méthyle, le méthacrylate de méthyle, le méthacrylate d'hydroxyéthyle, l'acrylate d'hydroxyéthyle, l'acide acrylique, l'acide méthacrylique, l'acrylate de glycéryle, le méthacrylate de glycéryle, le méthacrylamide, et l'acrylamide, les vinyles sont choisis parmi l'éthylène, le propylène, le styrène, le chlorure de vinyle, l'acétate de vinyle, la vinylpyrrolidone, et le difluorure de vinylidène, les nylons sont choisis parmi le polycaprolactame, le polylauryl lactame, le polyhexaméthylène adipamide, et le polyhexaméthylène dodécanediamide, les matériaux organiques sont choisis parmi le tissu humain, le bois, la cellulose, le carbone comprimé, et le caoutchouc, les métaux sont choisis parmi le titane, l'acier inoxydable, le cobalt-chrome, l'or, l'argent, le cuivre, le platine et leurs alliages, et les céramiques sont choisies parmi le nitrure de silicium, le carbure de silicium, le zirconium, et l'alumine, y compris les combinaisons de tels biomatériaux.

42. Procédé selon l'une des revendications 11 ou 22, dans lequel le dispositif est choisi dans le groupe constitué par les dispositifs vasculaires, les dispositifs orthopédiques, les dispositifs dentaires, les dispositifs de libération de médicament, les dispositifs ophtalmiques, les déviations du drain du glaucome, les dispositifs urologiques, les prothèses synthétiques, le tubage et les membranes de la dialyse, le tubage et les membranes de l'oxygénateur du sang, les poches de sang, les sutures, les membranes, les dispositifs de culture cellulaire, les matériaux du support chromatographique, et les biocapteurs.

43. Procédé selon la revendication 42, dans lequel les dispositifs vasculaires sont choisis parmi les greffons, les stents, les cathéters, les valves, les coeurs artificiels, et les dispositifs d'assistance cardiaque, les dispositifs orthopédiques sont choisis parmi les implants de jointure, les dispositifs de réparation de fracture, et les tendons artificiels, les dispositifs dentaires sont choisis parmi les implants dentaires, et les dispositifs de réparation de fracture, et les dispositifs urologiques sont choisis parmi le pénien, le sphincter, l'urètre, la vessie et les dispositifs rénaux.

44. Composition selon l'une quelconque des revendications 1-9 et 39, dans laquelle l'agent bioactif est un agent d'anti-prolifération.

45. Composition selon l'une quelconque des revendications 1-9 et 39, dans laquelle l'agent bioactif est le β-oestradiol.

46. Procédé selon l'une quelconque des revendications 11-24, 26, 28, dans lequel l'agent bioactif est un agent d'anti-prolifération.

47. Procédé selon l'une quelconque des revendications 11-24, 26, 28, dans lequel l'agent bioactif est le β-oestradiol.

48. Combinaison selon l'une quelconque des revendications 30-38, dans laquelle l'agent bioactif est un agent d'anti-prolifération.

49. Combinaison selon l'une quelconque des revendications 30-38, dans laquelle l'agent bioactif est le β-oestradiol.
